# FASCICULE DE BREVET EUROPEEN

(11) **EP 0 716 841 B1**
(45) Date de publication et mention de la délivrance du brevet: **08.03.2000**
(21) Numéro de dépôt: 95420358.4
(22) Date de dépôt: 12.12.1995
(51) Int. Cl.: A61F 2/44

(54) **Prothèse discale élastique**
Elastische Bandscheibenprothese
Elastic disc prosthesis

(30) Priorité: 16.12.1994 FR 9415435
(43) Date de publication de la demande: 19.06.1996
(73) Titulaire: TORNIER SA, 38330 Saint-Ismier (FR)
(72) Inventeur: Ratron, Yves-Alain, F-38000 Grenoble (FR)
(74) Mandataire: Myon, Gérard Jean-Pierre

(56) Documents cités:
- EP-A- 0 346 269
- EP-A- 0 353 936
- EP-A- 0 538 183
- DE-A- 2 263 842

## Description

La présente invention a trait à une prothèse discale élastique destinée à remplacer totalement ou partiellement le disque endommagé entre deux vertèbres d'une colonne vertébrale.

On connaît des prothèses de ce genre qui sont constituées de deux plateaux munis chacun d'ailerons d'ancrage et d'orifices filetés pour leur fixation dans les vertèbres après ablation du disque endommagé. Les deux plateaux sont séparés par un organe d'articulation constitué d'une calotte sphérique à base cylindrique. L'un des plateaux comporte une portée sphérique de même diamètre que la calotte, tandis que l'autre plateau est prévu pour recevoir la base cylindrique pour le maintien axial de ladite calotte.

Cette prothèse discale assure uniquement les déplacements axiaux des vertèbres l'une par rapport à l'autre, mais elle ne permet pas d'amortir les déplacements verticaux lorsque les vertèbres sont en charge. En outre, elle présente l'inconvénient d'être introduite et posée par voie antérieure, ce qui engendre par la suite des difficultés et des gênes pour le patient.

EP-A-0 538 183 divulgue une prothèse discale comprenant des lamelles transversales réparties autour de zones de fixation et séparées par des fentes. Ces lamelles sont essentiellement rectilignes et parallèles aux plateaux de la prothèse et seul un faible écrasement de la prothèse peut être obtenu.

C'est à ces inconvénients qu'entend plus particulièrement remédier la présente invention.

La prothèse discale suivant l'invention comprend deux plateaux opposés reliés entre eux par des cloisons susceptibles de se déformer élastiquement suivant la charge appliquée sur la prothèse.

Le premier plateau de la prothèse discale suivant l'invention comprend une cloison verticale qui se prolonge respectivement de chaque côté de son axe principal par des cloisons courbes qui sont solidaires du second plateau pour délimiter un espace libre dont le volume est susceptible de varier suivant la charge appliquée.

Le dessin annexé, donné à titre d'exemple, permettra de mieux comprendre l'invention, les caractéristiques qu'elle présente et les avantages qu'elle est susceptible de procurer :

Fig. 1 est une vue en perspective illustrant la prothèse de disque intervertébral suivant la présente invention.

Fig. 2 et 3 sont des vues montrant la mise en place de la prothèse discale entre deux vertèbre adjacentes d'une colonne vertébrale.

Fig. 4 est une vue de côté représentant la prothèse lorsque les vertèbres sont soumises à une charge modérée.

Fig. 5 est une vue de côté illustrant la prothèse lorsque les vertèbres sont soumises à une charge élevée.

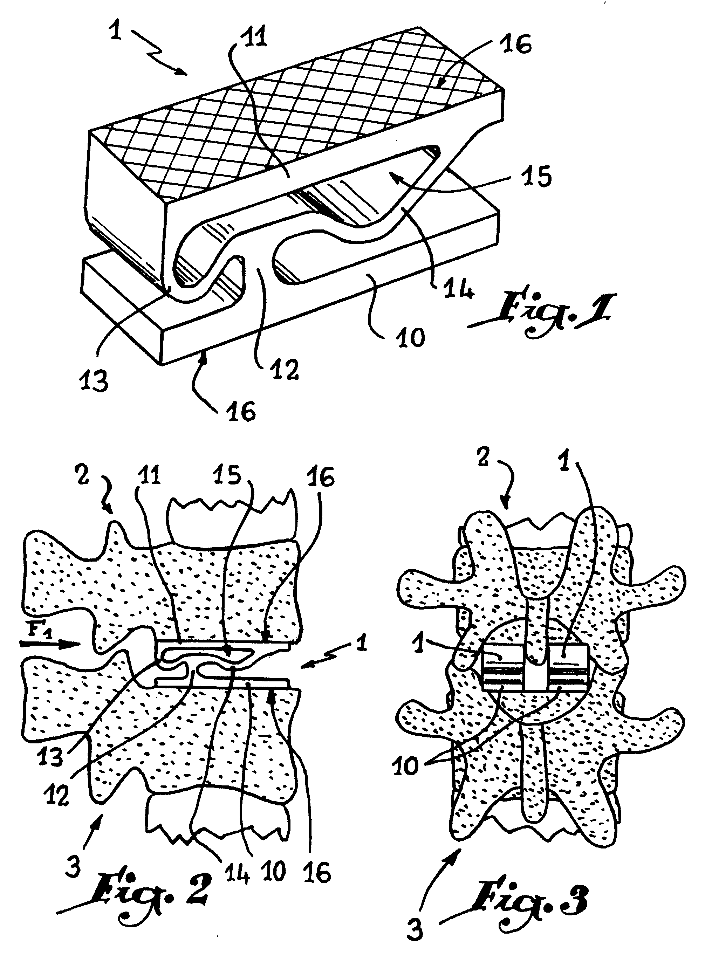

On a représenté en fig. 1 à 3 une prothèse discale 1 destinée à remplacer totalement ou partiellement un disque vertébral compris entre deux vertèbres adjacentes 2 et 3.

La prothèse discale 1 comprend deux plateaux opposés 10 et 11 qui sont disposés dans deux plans horizontaux parallèles lorsqu'elle est au repos, c'est-à-dire lorsqu'elle ne reçoit pas de charge, ni modérée, ni élevée.

Le premier plateau 10 comporte une cloison verticale 12 de faible hauteur qui est décalée latéralement par rapport au milieu dudit plateau. La cloison verticale 12 se prolonge respectivement de chaque côté de son axe principal par des cloisons 13, 14 qui sont solidaires du second plateau 11.

Les cloisons 13 et 14 présentent des profils courbes de rayons différents, de manière à délimiter avec le plateau 11 un espace libre 15.

La courbure de chaque cloison 13 et 14 est convexe vers le bas, c'est-à-dire en direction du plateau 10.

Les faces des plateaux 10 et 11 qui sont en contact avec les vertèbres 2 et 3 sont recouvertes d'aspérités 16 destinées à assurer un ancrage adéquat dans le tissu osseux.

La conformation des cloisons 12, 13 et 14 autorise le déplacement élastique du plateau 10 par rapport au plateau 11 selon la charge appliquée d'un des plateaux sur L'autre.

La prothèse discale 1 est constituée d'une pièce monobloc réalisée en matière métallique suffisamment élastique pour amortir les différentes charges soumises aux vertèbres.

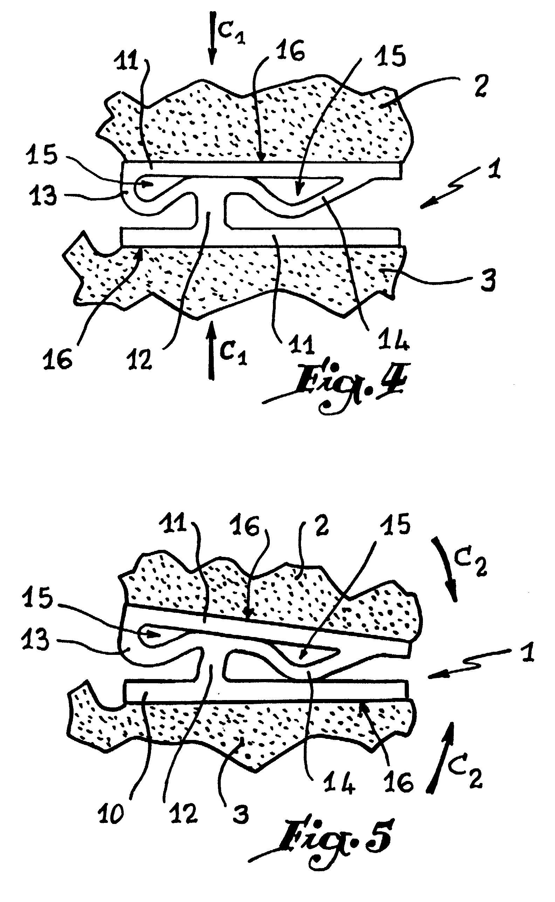

On note que le traitement chirurgical de remplacement d'un disque vertébral situé entre les deux vertèbres 2 et 3 impliquera généralement la pose de deux prothèses discales 1 suivant la présente invention, une de part et d'autre du canal spinal (fig. 2 et 3). Les prothèses discales 1 sont introduites entre les deux vertèbres 2 et 3 par voie postérieure suivant la flèche F1 comme représenté en fig. 2.

En fig. 4, on a représenté la prothèse discale 1 lorsque les vertèbres 2 et 3 sont soumises à une charge modérée. On constate que sous l'effet de la charge C1, les cloisons 13 et 14 subissent une déformation élastique amenant l'extrémité libre de la cloison 12 en appui contre le plateau 11. La rigidité ou la souplesse de cette déformation est contrôlée par la forme et l'épaisseur des cloisons 13 et 14, ainsi que par la nature de la matière choisie. La déformation élastique des cloisons 12 et 14 entraîne une réduction importante du volume de l'espace libre 15, permettant d'amortir La charge C₁.

En fig. 5, on a représenté La prothèse discale 1 lorsque les vertèbres 2 et 3 sont soumises à une charge plus élevée C2. On constate qu'une fois l'espace libre 15 complètement réduit, c'est-à-dire fermé en deux espaces distincts, la cloison verticale 12 commence à se déformer latéralement, entraînant un mouvement élastique complémentaire des deux plateaux 10 et 11. Ce mouvement élastique complémentaire consiste en un basculement des deux plateaux 10 et 11 de manière que la cloison 14 et plus particulièrement son profil arrondi vienne en appui sur le plateau 10.

Le centre de rotation de ce basculement, la rigidité ou la souplesse de cette déformation sont contrôlées par la forme et l'épaisseur de la cloison verticale 12.

## Revendications

1. Prothèse discale intervertébrale comprenant deux plateaux opposés reliés entre eux par des cloisons disposées dans des plans différents de ceux des deux plateaux (10 et 11) et susceptibles de se déformer élastiquement suivant la charge appliquée sur la prothèse, le premier plateau (10) comportant une cloison verticale (12) qui se prolonge respectivement de chaque côté de son axe principal par des cloisons (13 et 14) à profils courbes qui sont solidaires du second plateau pour délimiter avec lui un espace libre (15) dont le volume est susceptible de varier suivant la charge appliquée, de manière que la prothèse (1) présente des raideurs différentes en fonction de la charge appliquée.

2. Prothèse suivant la revendication 1, caractérisée en ce que la cloison verticale (12) est décalée latéralement par rapport au milieu du premier plateau (10).

3. Prothèse suivant la revendication 1, caractérisée en ce que les cloisons (13 et 14) présentent des profils courbes de rayons différents.

4. Prothèse suivant la revendication 1, caractérisée en ce qu'elle est réalisée dans une matière métallique suffisamment élastique pour amortir les différentes charges soumises aux vertèbres (2 et 3).

5. Prothèse suivant la revendication 1, caractérisée en ce que la face des plateaux (10 et 11) recevant les vertèbres (2 et 3) sont recouvertes d'aspérités (16) destinées à assurer un ancrage adéquat dans les tissus osseux.

6. Prothèse suivant la revendication 4, caractérisée en ce que la courbure des cloisons (13 et 14) est tournée du côté du plateau (10).

7. Prothèse selon l'une des revendications précédentes, caractérisée en ce que lesdites cloisons (13 et 14) à profils courbes sont aptes à subir une déformation élastique sous charge (C₁) amenant l'extrémité libre de ladite cloison verticale (12) en appui contre ledit second plateau (11).

8. Prothèse selon la revendication 7, caractérisée en ce que ladite cloison verticale (12) est apte à se déformer latéralement, sous une charge (C₂) plus élevée que celle (C₁) entraînant ladite déformation élastique desdites cloisons (13 et 14) à profils courbes, entraînant un mouvement élastique complémentaire desdits plateaux (10 et 11).

9. Prothèse selon la revendication 8, caractérisée en ce que ledit mouvement élastique complémentaire consiste en un basculement desdits deux plateaux (10 et 11), tel que l'une desdites cloisons (13 et 14) à profils courbes peut venir en appui sur ledit premier plateau (10).

## Patentansprüche

1. Bandscheibenprothese bestehend aus zwei gegenüberliegenden Platten, die über Innenwände in Verbindung stehen, die in zu den Ebenen der zwei Platten (10,11) unterschiedlichen Ebenen angeordnet und entsprechend der auf die Prothese einwirkenden Belastung elastisch verformbar sind, wobei die erste Platte (10) einen vertikalen Wandsteg (12) aufweist, der sich zu beiden Seiten seiner Hauptachse durch die Innenwände (13,14) mit gekrümmten Profilen verlängert, die mit der zweiten Platte fest verbunden sind, um mit dieser einen Freiraum (15) einzuschließen, dessen Volumen entsprechend der einwirkenden Belastung veränderlich ist derart, daß die Prothese (1) in Abhängigkeit der einwirkenden Belastung unterschiedliche Steifigkeiten aufweist.

2. Prothese nach Anspruch 1,
dadurch **gekennzeichnet,**
daß der vertikale Wandsteg (12) seitlich zur Mitte der ersten Platte (10) versetzt ist.

3. Prothese nach Anspruch 1,
dadurch **gekennzeichnet**,
daß die Innenwände (13,14) gekrümmte Profile mit unterschiedlichen Radien aufweisen.

4. Prothese nach Anspruch 1,
dadurch **gekennzeichnet,**
daß diese aus einem weitgehend elastischen Metallwerkstoff hergestellt ist, um die auf die Wirbel (2,3) einwirkenden unterschiedlichen Belastungen aufzufangen.

5. Prothese nach Anspruch 1,
dadurch **gekennzeichnet**,
daß die die Wirbel (2,3) aufnehmende Fläche der Platten (10,11) mit Unebenheiten (16) überzogen ist, die eine hinreichende Verankerung im Knochengewebe sicherstellen.

6. Prothese nach Anspruch 4,
dadurch **gekennzeichnet,**
daß die Krümmung der Innenwände (13,14) in Richtung zur Platte (10) gebogen ist.

7. Prothese nach einem der vorhergehenden Ansprüche,
dadurch **gekennzeichnet,**
daß die Innenwände (13,14) mit gekrümmten Profilen geeignet sind, eine elastische Verformung unter einer Belastung (C₁) durchzuführen, die das freie Ende des vertikalen Wandstegs (12) gegen die zweite Platte (11) zum Anschlag kommen läßt.

8. Prothese nach Anspruch 7,
dadurch **gekennzeichnet**,
daß der vertikale Wandsteg (12) geeignet ist, sich unter einer Belastung (C₂) seitlich zu verformen, die größer ist als die Belastung (C₁), die die elastische Verformung der Innenwände (13,14) mit gekrümmten Profilen nach sich zieht, wodurch eine entsprechende elastische Bewegung der Platten (10,11) herbeigeführt wird.

9. Prothese nach Anspruch 8,
dadurch **gekennzeichnet,**
daß diese entsprechende elastische Bewegung in einem Kippen dieser zwei Platten (10,11) besteht derart, daß eine der Innenwände (13,14) mit gekrümmten Profilen auf der ersten Platte (10) zur Auflage kommt.

## Claims

1. Intervertebral disc prosthesis comprising two opposed plates joined to one another by partitions disposed in different planes from those of the two plates (10 and 11) and adapted to be elastically deformed according to the load applied on the prosthesis, the first plate (10) having a vertical partition (12) which extends respectively on each side of its principal axis by way of partitions (13 and 14) with curved profiles which are rigidly joined to the second plate so as to delimit with the latter a free space (15) the volume of which is adapted to vary according to the load applied, such that the prosthesis (1) presents different degrees of resistance to elastic deformations in dependence on the load applied.

2. Prosthesis according to claim 1, characterised in that the vertical partition (12) is laterally offset with respect to the middle of the first plate (10).

3. Prosthesis according to claim 1, characterised in that the partitions (13 and 14) present curved profiles of different radii.

4. Prosthesis according to claim 1, characterised in that it is made from a metallic material with sufficient elasticity to cushion the various loads to which the vertebrae (2 and 3) are subjected.

5. Prosthesis according to claim 1, characterised in that the surfaces of the plates (10 and 11) that receive the vertebrae (2 and 3) are roughened all over (16) in order to provide adequate anchorage in the bony tissues.

6. Prosthesis according to claim 4, characterised in that the curvature of the partitions (13 and 14) faces away from the plate (10).

7. Prosthesis according to any one of the preceding claims, characterised in that said partitions (13 and 14) with curved profiles are capable of withstanding an elastic deformation under load (C₁) that brings the free end of said vertical partition (12) to rest against said second plate (11).

8. Prosthesis according to claim 7, characterised in that said vertical partition (12) is capable of being deformed laterally under a greater load (C₂) than that (C₁) giving rise to said elastic deformation of said partitions (13 and 14) with curved profiles, giving rise to a complementary elastic movement by said plates (10 and 11).

9. Prosthesis according to claim 8, characterised in that said complementary elastic movement consists of a rocking motion of said two plates (10 and 11), such that one of said partitions (13 and 14) with curved profiles is able to come to rest against said first plate (10).
